## Europäisches Patentamt

(19) **European Patent Office**

**Office européen des brevets**

(11) Veröffentlichungsnummer: **0 152 887**
**B1**

(12) **EUROPÄISCHE PATENTSCHRIFT**

(45) Veröffentlichungstag der Patentschrift:
**18.10.89**

(51) Int..Cl.⁴: **A 61 K 6/10**, C 08 K 9/04,
C 08 L 83/04

(21) Anmeldenummer: **85101411.8**

(22) Anmeldetag: **11.02.85**

(54) Modifizierte Füllstoffe für Silikonpasten, deren Verwendung und diese Füllstoffe enthaltende Dentalabformmassen.

(30) Priorität: **21.02.84 DE 3406233**

(43) Veröffentlichungstag der Anmeldung:
**28.08.85 Patentblatt 85/35**

(45) Bekanntmachung des Hinweises auf die Patenterteilung:
**18.10.89 Patentblatt 89/42**

(84) Benannte Vertragsstaaten:
**AT BE CH DE FR GB IT LI LU NL SE**

(56) Entgegenhaltungen:
**AT-B- 209 484**
**AT-B- 252 564**
**US-A- 3 591 519**
**US-A- 4 007 153**

(73) Patentinhaber: **BAYER AG,**
**D-5090 Leverkusen 1 Bayerwerk (DE)**

(72) Erfinder: **Schwabe, Peter, Dr., Dudweiler Strasse 17,**
**D-5090 Leverkusen 1 (DE)**
Erfinder: **Voigt, Reiner, Dipl.-Ing.,**
**Gustav-Radbruch-Strasse 14, D-5090 Leverkusen 3 (DE)**

## Beschreibung

Die vorliegende Erfindung betrifft paraffinbeladene Füllstoffe für Silikon-Pasten, insbesondere für knetbare Massen zur Herstellung genauer Abformungen von Zähnen. Bei den Silikon-Pasten handelt es sich vorzugsweise um an sich bekannte kaltvulkanisierende Zweikomponenten-Silikonkautschuksysteme, bei denen zwei Pasten vermengt werden, die dann nach ca. 2–5 Minuten bei Raumtemperatur vernetzen.

Silikon-Pasten zur Zahnabformung sind weit verbreitet. (vgl. US-A-4 007 153, AT-A-252 564, AT-A-209 484). Im allgemeinen bestehen sie aus einem mit Füllstoffen vermischten Silikonöl auf Basis eines endständige Hydroxylgruppen aufweisenden Polydimethylsiloxans, welches je nach Applikationsmethode in verschiedenen Konsistenzen erhältlich ist, und einer flüssigen oder pastenförmigen Härterkomponente, die ein Metallsalz einer Monocarbonsäure als Katalysator und einen Kieselsäureester als Vernetzer enthält.

Diese beiden Komponenten werden vor der Anwendung miteinander gemischt und vernetzen bei Raumtemperatur innerhalb von 2–5 Minuten infolge einer Polykondensationsreaktion. Hierbei entstehen neben dem vernetzten Silikongummi noch geringe Mengen Alkohol, die langsam aus dem Gummi heraus diffundieren und eine lineare Schrumpfung hervorrufen, was bei den Abformungen zu Ungenauigkeiten führt.

Wesentlich geringer ist die lineare Schrumpfung bei den erst seit wenigen Jahren bekannten Vinylsilikon-Abformmassen. Diese Massen bestehen aus zwei Pasten, einer Silikonöl, Füllstoff und Vernetzer enthaltenden Basispaste und einer Silikonöl, Füllstoff und Katalysator enthaltenden Katalysatorpaste.

Bei dem Silikonöl handelt es sich hierbei um ein endständige Vinylgruppen aufweisendes Polydimethylsiloxan, der Vernetzer enthält die reaktiven SiH-Gruppen und der Katalysator besteht aus einem Platinkomplex. Zu der größeren Dimensionsgenauigkeit des Modells kommt bei diesem System noch die bessere Dosierbarkeit von Basis- und Katalysatorpaste infolge gleicher Pasten-Viskosität und dem abgestimmten Mischungsverhältnis von 1 : 1 der beiden Pasten hinzu, sowie die völlige Geschmack- und Geruchlosigkeit der Pasten.

Da in der Zahnheilkunde verschiedene Methoden zur Abformung bezahnter, teilbezahnter und unbezahnter Kiefer und der Schleimhaut angewendet werden, ist ein Angebot von Abformmassen in verschiedenen Viskositätklassen notwendig, z. B. niedrig-, mittel-, hochviskose und knetbare Massen. Diese Massen bestehen jeweils aus einer Basispaste und einer speziell auf die erforderliche Verarbeitungszeit eingestellten Katalysatorpaste.

Während bei den niedrig-, mittel- und hochviskosen Massen die Basis- und die Katalysatorpaste aus Tuben oder Kartuschen in gleichlangen Strängen auf einen Anmischblock ausgedrückt und mit einem Spachtel vermischt werden, entnimmt man die Basis- und Katalysatorpaste der knetbaren Abformmasse mit entsprechend gekennzeichneten Dosierlöffeln aus Kunststoffdosen oder -bechern und verknetet die gleichgroßen Klumpen mit den Fingern zu einer homogenen Masse. Diese Masse wird in einen Abformlöffel gegeben und dann im Mund des Patienten plaziert. Nach einigen Minuten kann der Löffel mit der zu Gummi vernetzten Masse aus dem Mund des Patienten entfernt werden. Die negative Form der entsprechenden Kiefersituation wird danach noch durch Einsatz von niedrigviskoser Abformmasse korrigiert und dann mit einem wäßrigen Gipsbrei ausgegossen, der im erhärteten Zustand als Gipsmodell die Kiefersituation wiedergibt.

Bei der Entnahme der Basis- und Katalysatorpasten und auch beim Mischen der Pasten durch Kneten mit den Fingern ist es wichtig, daß die Masse nicht an den Entnahmelöffeln bzw. den Fingern klebt und/oder Rückstände hinterläßt. Das Kleben kann man durch Auswahl geeigneter Füllstoffe und durch Zusatz von Paraffinöl verhindern. Während man bei den kondensationsvernetzenden Silikonabformmassen den Klebeeffekt mit Talkum teilweise abdecken kann, ist dies bei den additionsvernetzenden Vinylsilikon-Abformmassen nicht möglich, da Talkum das Abbindeverhalten der Massen negativ beeinflußt. Der Zusatz von Paraffinöl ist auch nicht ganz unproblematisch. Einerseits benötigt man eine Menge von 6–8 Gewichtsprozent in der Masse, um das Kleben zu verhindern, andererseits schwitzen sowohl die Pasten als auch die Abformungen während der Lagerung einen Teil des Paraffinöles aus, d. h. in den Vertiefungen der Pastenoberfläche sammelt sich Paraffinöl an bzw. auf der Oberfläche der Abformungen bilden sich Tropfen von Paraffinöl. Letztere können zum einen den Verbund zwischen der ausgehärteten knetbaren Masse und dem niedrigviskosen Korrekturmaterial verhindern, falls die Korrektur erst nach einem längeren Zeitraum vorgenommen wird, und zum anderen Fehlstellen auf dem Gipsmodell ergeben. Außerdem verringert der Paraffinölzusatz die notwendige Adhäsion der Masse an den Wandungen der Abformlöffel.

Diese Probleme werden erfindungsgemäß durch den Einsatz von paraffinbeladenen Füllstoffen in den Pasten vermieden. Um die Klebrigkeit der Pasten zu eliminieren, benötigt man vorzugsweise nur noch etwa 4–5 Gewichtsprozent Paraffinöl in der Paste, wovon sich jedoch etwa 1–2 Gewichtsprozent Paraffinöl bereits auf der Oberfläche der Füllstoffe befinden. Somit enthalten die Pasten bevorzugt nur noch etwa 2–4 Gewichtsprozent freies Paraffinöl, welches wiederum Affinität zum paraffinbeladenen Füllstoff besitzt und weniger zum Ausschwitzen neigt. Man erhält auf diese Weise weiche, geschmeidige und lagerstabilere Massen bzw. Abformungen, d. h. es bilden sich kaum noch Tropfen von Paraffinöl in der Paste wie auch auf dem Gummi der Abformung, so daß auch nach längerer Lagerung der Abformung eine einwandfreie Korrekturabformung durchgeführt und/ oder ein einwandfreies Gipsmodell hergestellt werden kann.

Außerdem ist die Haftung der Masse an den Wandungen der Abformlöffel verbessert.

Gegenstand der Erfindung ist somit ein anorganischer Füllstoff für pastenförmige Silikonmassen, dadurch gekennzeichnet, daß der Füllstoff aus Calciumcarbonat, Cristobalit oder Quarzmehl oder Mischungen daraus besteht, wobei der mittlere Teilchendurchmesser der Füllstoffteilchen zwischen 1 und 25 μm liegt und die Oberfläche der Füllstoffteilchen mit 0,5–5 Gew.-%, bezogen auf den gesamten Füllstoff, mit Paraffinöl einer Viskosität von 120–300 mPa. · s bei 20 °C beladen ist.

Vorzugsweise enthalten die Silikonpasten 30 bis 90 Gew.-%, insbesondere 50 bis 80 Gew.-%, der erfindungsgemäßen modifizierten Füllstoffe (bezogen auf Gesamtgewicht der Paste), gegebenenfalls im Gemisch mit konventionellen nichtmodifizierten Füllstoffen.

Vorzugsweise werden die erfindungsgemäßen modifizierten Füllstoffe in bei Raumtemperatur härtbaren Dentalmassen auf Polysiloxanbasis verwendet. Man unterscheidet dabei, wie schon eingangs ausgeführt, additionsvernetzende und kondensationsvernetzende Systeme. Als wesentliche Komponenten enthalten nach dem Additionssystem vernetzende Pasten

a) Organopolysiloxane mit zwei oder mehr Vinylgruppen im Molekül

b) hydrophobe anorganische Füllstoffe gemäß Erfindung, gegebenenfalls zusammen mit konventionellen anorganischen Füllstoffen,

c) Organohydrogenpolysiloxane als Vernetzer,

d) einen Katalysator zur Beschleunigung der Additionsreaktion,

e) Paraffinöl und

f) Farbstoffe.

Die nach dem Kondensationssystem mit einer flüssigen oder pastenförmigen Härterkomponente, die aus einem Metallsalz einer Monocarbonsäure als Katalysator und einem Kieselsäureester als Vernetzer besteht, vernetzenden Pasten enthalten als wesentliche Bestandteile

g) Organopolysiloxane mit zwei oder mehr Hydroxylgruppen in Molekül,

h) Füllstoffe gemäß Erfindung, gegebenenfalls in Kombination mit konventionellen anorganischen Füllstoffen,

i) Paraffinöl und

j) Farbstoffe.

Die erfindungsgemäße Füllstoffe enthaltenden knetbaren Silikonpasten zeichnen sich durch ihre Lagerstabilität und Klebfreiheit beim Vermischen von Basis- und Katalysatorpaste bzw. von Paste und Härterkomponente aus. Sie eignen sich für die Herstellung genauer Abformungen von Zähnen wegen ihrer detailgetreuen Wiedergabe im Gipsmodell, nachdem die Vernetzer- und Katalysatorpaste gut gemischt in die Mundhöhle eingebracht und darin verfestigt wurde, die Abformung mit einer Gipsaufschlämmung ausgegossen und zu einem Modell ausgehärtet worden ist. Dieses Ergebnis wird erzielt, weil sich auf der Oberfläche der Abformung keine Tropfen von Paraffinöl bilden, die die Verbindung zwischen der Vorabformung aus den knetbaren Massen und der Korrekturabformung aus niedrigviskosen Massen stören und/oder die Oberfläche des Gipsmodells verfälschen.

Die Einsatzstoffe der oben genannten bei Raumtemperatur härtbaren Pasten sind an sich bekannt.

Bei dem Silikonöl (a) handelt es sich um ein vinylendgestopptes Polydimethylsiloxan, dessen Viskosität bevorzugt im Bereich von 500 bis 5 000 000 mPa · s bei 20 °C liegt.

Wie bereits angegeben eignen sich als Füllstoffe (b) modifizierte Quarz- und Cristobalitmehle und Calciumcarbonat. Die Teilchengröße der Füllstoffe liegt vorzugsweise zwischen 1 und 25 μm. Zu feinteilige Füllstoffe können zu einer unerwünschten Strukturviskosität der Paste führen.

Der Vernetzer (c) ist ein Polydimethylsiloxan, welches in seinem Molekül Wasserstoffatome an mindestens zwei Siliciumatomen aufweist.

Bei dem Katalysator (d) handelt es sich z. B. um einen Platinkomplex, der aus Hexachloroplatin-(IV)-säure hergestellt wurde. Auch diese Verbindungen sind an sich bekannt.

Das Paraffinöl (e) besteht aus einem bei Raumtemperatur flüssigen Gemisch von Alkanen mit einer Viskosität von 120–300 mPa · s, besonders bevorzugt 170–230 mPa · s, bei 20 °C.

Farbstoffe (f) werden zur Unterscheidung der Basis- und Katalysatorpaste und zur Mischkontrolle eingesetzt. Anorganische und organische Farbpigmente werden bevorzugt.

Bei dem Silikonöl (g) handelt es sich um ein hydroxylendgestopptes Polydimethylsiloxan, dessen Viskosität vorzugsweise im Bereich von 500 bis 200 000 mPa · s bei 20 °C liegt.

Die Füllstoffe (h), das Paraffinöl (i) und die Farbstoffe (j) sind dieselben Substanzen wie oben unter (b), (e) und (f) beschrieben.

Die Beladung der Füllstoffe (b) bzw. (h) mit Paraffinöl erfolgt in an sich bekannten Mahlvorrichtungen, bevorzugt in Kugelmühlen, deren Wände z. B. mit Aluminiumoxid beschichtet sind und deren Kugeln z. B. aus Aluminiumoxid bestehen (je nach Härte der zu beladenden Füllstoffe). Man füllt den Füllstoff in den Kugelmühlenbehälter und gibt 0,5–5, vorzugsweise 1–2,5 Gew.-% Paraffinöl und die Kugeln zu. Den verschlossenen Behälter läßt man auf einem Rollwerk rotieren. Selbstverständlich kann der Mahlvorgang auf einer geeigneten Apparatur aber auch kontinuierlich erfolgen. Danach wird der mit Paraffinöl beschichtete rieselfähige Füllstoff durch Windsichtung fraktioniert. Die Menge des zuzusetzenden Paraffinöles ist begrenzt, da oberhalb der angegebenen Grenze der Füllstoff zusammenklumpt und sich nicht mehr einwandfrei windsichten läßt.

Die nachfolgenden Beispiele, in denen alle Teile Gewichtsteile bedeuten, erläutern die Erfindung.

Beispiel 1 (Vergleichsversuch)

Die Basispaste wurde hergestellt durch Vermischen in einem Kneter von 175 Teilen vinylendgestopptem Polydimethylsiloxan mit einer Viskosität von 80 000 mPa · s bei 20 °C, 50 Teilen dimethylhydrogensilylendgestopptem Polydimethylsiloxan mit einer Viskosität von 50 mPa · s bei 25 °C, 650 Teilen Quarz-Feinstmehl mit einer mittleren Teilchengröße von ca. 4 μm, 60 Teilen Calciumcarbonat mit einer mittleren Teilchengröße von ca. 8 μm, 60 Teilen Paraffinöl mit einer Viskosität von ca. 180 mPa · s bei 20 °C und 5 Teilen anorganischem Farbpigment.

Die Herstellung der Katalysatorpaste erfolgte durch Vermischen in einem Kneter von 229,8 Teilen vinylendgestopptem Polydimethylsiloxan mit einer Viskosität von 80 000 mPa · s bei 20 °C, 650 Teilen Quarz-Feinstmehl mit einer mittleren Teilchengröße von ca. 4 μm, 60 Teilen Calciumcarbonat mit einer mittleren Teilchengröße von ca. 8 μm, 60 Teilen Paraffinöl mit einer Viskosität von 180 mPa · s und 0,2 Teilen eines Komplexes aus Platin und Dimethylvinyl-disiloxan.

Beide Pasten sind geschmeidig, klebfrei und lassen sich gut kneten, bei einer Lagerung von 7 Tagen bilden sich jedoch in den Vertiefungen der beiden Pastenoberflächen Ansammlungen von Paraffinöl.

15 g Basispaste und 15 g Katalysatorpaste wurden mit den Fingern in 30 Sekunden zu einer homogenen Masse verknetet, auf einen Abformlöffel und unter geeignetem Druck in den Mund gebracht. Innerhalb von 5 Minuten härtete die Masse zu einem Elastomeren aus. Nach Entnahme aus dem Mund, Abwaschen mit fließendem Wasser und Abtupfen mit Zellstoff wurde die Abformung 24 Stunden bei Raumtemperatur gelagert. An der Oberfläche der Abformung hatten sich danach Tröpfchen von Paraffinöl gebildet, welche sich auf das Gipsmodell, hergestellt durch Ausgießen der Abformung mit einer Gipsaufschlämmung von 100 Teilen Calciumsulfat-Hemihydrat und 30 Teilen Wasser und anschließender Lagerung über 30 Minuten, in Form von Vertiefungen übertrugen und somit das Gipsmodell verfälschten.

In eine wie vorbeschrieben hergestellte, 24 Stunden gelagerte und mit Öltropfen versehene Abformung wurde eine niedrigviskose Vinylsilikon-Abformmasse zur Korrekturabformung gegeben und unter geeignetem Druck in den Mund gebracht. Die nach 5 Minuten zu einem Elastomeren ausgehärtete Masse hatte nach dem Entfernen aus dem Mund an den ölbenetzten Stellen keine Haftung zu dem Vorabformmaterial.

Beispiel 2 (erfindungsgemäß)

In einer Kugelmühle werden 2000 Teile Quarzmehl und 30 Teile Paraffinöl mit einer Viskosität von 180 mPa · s bei 20 °C 60 Minuten vermischt und danach windsichtet, wovon die Fraktion unter 25 μm Korngröße für die Pastenherstellung verwendet wurde.

In einem Kneter wurde die Basispaste hergestellt durch Vermischen von 170 Teilen vinylendgestopptem Polydimethylsiloxan mit einer Viskosität von 80 000 mPa · s bei 20 °C, 50 Teilen dimethylhydrogensilylenendgestopptem Polydimethylsiloxan mit einer Viskosität von 50 mPa · s bei 20 °C, 742 Teilen des Paraffinöl-beladenen Quarz-Feinstmehls, 33 Teilen Paraffinöl mit einer Viskosität von 180 mPa · s bei 20 °C und 5 Teilen an organischem Farbpigment.

Die Katalysatorpaste wurde in einem Kneter hergestellt durch Vermischen von 230 Teilen vinylendgestopptem Polydimethylsiloxan mit einer Viskosität von 80 000 mPa · s bei 20 °C, 732 Teilen des mit Paraffinöl beladenen Quarz-Feinstmehls, 37,8 Teilen Paraffinöl mit einer Viskosität von 180 mPa · s bei 20 °C und 0,2 Teilen Platin-Siloxan-Komplex.

Beide Pasten waren geschmeidig und klebfrei und ließen sich gut kneten. Nach einer Lagerung von 2 Monaten konnte keine Paraffinöl-Ausscheidung festgestellt werden.

Die beiden Pasten wurden 1 : 1 gemischt und eine Abformung hergestellt, die nach 24 Stunden keine Öltröpfchen an der Oberfläche aufwies. Das Korrekturmaterial haftete einwandfrei und das Gipsmodell wies keine Fehler auf.

Beispiel 3 (Vergleichsversuch)

In einem Kneter wurde eine Paste hergestellt durch Vermischen von 210 Teilen hydroxylendgestopptem Polydimethylsiloxan mit einer Viskosität von 50 000 mPa · s bei 20 °C, 80 Teilen Paraffinöl mit einer Viskosität von 180 mPa · s bei 20 °C, 100 Teilen Calciumcarbonat, 6000 Teilen Quarzfeinstmehl von Beispiel 1 und 10 Teilen Titandioxid.

Die Paste war geschmeidig, klebfrei und ließ sich gut kneten. Bei einer Lagerung von 7 Tagen bildeten sich jedoch in den Vertiefungen der Pastenoberfläche Ansammlungen von Paraffinöl.

25 g Paste und 1 g einer Härterkomponente, bestehend aus Dibutylzinndilaurat und Tetraethoxysilan (1 : 1), wurden in 30 Sekunden zu einer homogenen Masse verknetet, auf einen Abformlöffel und unter geeignetem Druck in den Mund gebracht. Innerhalb von 5 Minuten härtete die Masse zu einem Elastomeren aus. Nach Entnahme aus dem Mund, Abwaschen mit fließendem Wasser und Abtupfen mit Zellstoff wurde die Abformung 24 Stunden bei Raumtemperatur gelagert. An der Oberfläche der Abformung hatten sich danach Tröpfchen von Paraffinöl gebildet, welche sich auf das Gipsmodell, hergestellt durch Ausgießen der Abformung mit einer Gipsaufschlämmung von 100 Teilen Calciumsulfat-Hemihydrat und 30 Teilen Wasser und anschließender Lagerung über 30 Minuten, in Form von Vertiefungen übertrugen und somit das Gipsmodell verfälschten.

In eine wie oben beschrieben hergestellte, 24 Stunden gelagerte und mit Öltropfen versehene Abformung wurde eine niedrigviskose Silikon-Abformmasse zur Korrekturabformung gegeben und unter geeignetem Druck in den Mund gebracht. Die nach 5 Minuten zu einem Elastomeren ausgehärtete Masse hatte nach dem Entfernen aus dem Mund an den ölbenetzten Stellen keine Haftung zu dem Vorabformmaterial.

Beispiel 4 (erfindungsgemäß)

Eine Paste wurde in einem Kneter hergestellt durch Vermischen von 220 Teilen hydroxylendgestopptem Polydimethylsiloxan mit einer Viskosität von 50 000 mPa · s bei 20°C, 40 Teilen Paraffinöl mit einer Viskosität von 180 mPa · s, 100 Teilen Calciumcarbonat, 630 Teilen wie in Beispiel 2 beschrieben mit Paraffinöl oberflächlich beladenem Quarzfeinstmehl und 10 Teilen Titandioxid.

Die Paste war geschmeidig und klebfrei und ließ sich gut kneten. Nach einer Lagerung von 2 Monaten konnte keine Paraffinöl-Abscheidung festgestellt werden.

Nach dem Mischen der Paste mit der Härterkomponente aus Dibutylzinndilaurat und Tetraethoxysilan wurde eine Abformung hergestellt, die nach 24 Stunden keine Öltröpfchen an der Oberfläche aufwies. Das Korrekturmaterial haftete einwandfrei und das Gipsmodell wies keine Fehler auf.

**Patentansprüche**

1. Anorganischer Füllstoff für pastenförmige Silikonmassen, dadurch gekennzeichnet, daß der Füllstoff aus Calciumcarbonat, Cristobalit oder Quarzmehl oder Mischungen daraus besteht, wobei der mittlere Teilchendurchmesser der Füllstoffteilchen zwischen 1 und 25 µm liegt und die Oberfläche der Füllstoffteilchen mit 0,5–5 Gew.-%, bezogen auf den gesamten Füllstoff, mit Paraffinöl einer Viskosität von 120–300 mPa · s bei 20°C beladen ist.

2. Füllstoff nach Anspruch 1, dadurch gekennzeichnet, daß die Oberfläche der Füllstoffteilchen mit 1–2,5 Gew.% Paraffinöl beladen ist.

3. Füllstoff nach Anspruch 2, dadurch gekennzeichnet, daß die Viskosität des Paraffinöls 170–230 mPa · s bei 20°C beträgt.

4. Verwendung von modifizierten Füllstoffen nach Anspruch 1 bis 3 in knetbaren, bei Raumtemperatur aushärtenden Dentalmassen auf Silikonbasis.

5. Verwendung gemäß Anspruch 4 in additionsvernetzenden Systemen.

6. Verwendung gemäß Anspruch 4 in kondensationsvernetzenden Systemen.

7. Dentalabformmassen auf Silikonbasis, dadurch gekennzeichnet, daß sie modifizierte Füllstoffe gemäß Anspruch 1 bis 3 enthalten.

**Claims**

1. Inorganic filler for pasty silicone compositions, characterized in that the filler consists of calcium carbonate, cristobalite or quartz flour or mixtures thereof, the mean particle size of the filler particles being between 1 and 25 µm and the surface of the filler particles being laden with 0.5–5% by weight, based on the total filler, of paraffin oil which has a viscosity of 120–300 mPa · s at 20°C.

2. Filler according to Claim 1, characterized in that the surface of the filler particles is laden with 1–2.5% by weight of paraffin oil.

3. Filler according to Claim 2, characterized in that the paraffin oil has a viscosity of 170–230 mPa · s at 20°C.

4. Use of modified fillers according to Claim 1 to 3 in kneadable, room temperature-curable dental compositions based on silicone.

5. Use according to Claim 4 in systems which undergo crosslinking by addition.

6. Use according to Claim 4 in systems which undergo crosslinking by condensation.

7. Dental impression compositions based on silicone, characterized in that they contain modified fillers according to Claim 1 to 3.

**Revendications**

1. Charge inorganique pour masses pâteuses aux silicones, caractérisée en ce que la charge se compose de carbonate de calcium, cristobalite ou poussière de quartz ou de mélanges de ces substances, la dimension moyenne des particules de la charge étant comprise entre 1 et 25 µm et la surface des particules de la charge étant chargée de 0,5 à 5% en poids, par rapport à l'ensemble de la charge, d'huile de paraffine d'une viscosité de 120 à 300 mPa · s à 20°C.

2. Charge selon la revendication 1, caractérisée en ce que la surface des particules de charge est chargée de 1 à 2,5% en poids d'huile de paraffine.

3. Charge selon la revendication 2, caractérisée en ce que la viscosité de l'huile de paraffine est de 170 à 230 mPa · s à 20°C.

4. Utilisation de charges modifiées selon les revendications 1 à 3 dans des masses dentaires à base de silicones, malléables, durcissables à température ambiante.

5. Utilisation selon la revendication 4 dans des systèmes réticulant par addition.

6. Utilisation selon la revendication 4 dans des systèmes réticulant par condensation.

7. Masses de moulages dentaires à base de silicones, caractérisées en ce qu'elles contiennent des charges modifiées selon les revendications 1 à 3.